# EUROPÄISCHE PATENTSCHRIFT

(11) **EP 1 188 430 B1**
(45) Veröffentlichungstag und Bekanntmachung des Hinweises auf die Patenterteilung: **17.11.2004**
(21) Anmeldenummer: 01122271.8
(22) Anmeldetag: 18.09.2001
(51) Int. Cl.: A61K 7/09

(54) **Verfahren zum dauerhaften Verformen von menschlichen Haaren**
Process for permanent waving of human hair
Procédé de mise en forme permanente des cheveux humains

(30) Priorität: 19.09.2000 DE 10046253
(43) Veröffentlichungstag der Anmeldung: 20.03.2002
(73) Patentinhaber: KPSS-Kao Professional Salon Services GmbH, 64297 Darmstadt (DE)
(72) Erfinder: Grit, Mustafa, Dr., 64579 Gernsheim (DE); Wagner, Helmar R., 64291 Darmstadt (DE); Dubowoj, Polina, Aree Place Condominium Room 801, Klongton, Klongtoey, Bangkok 10110 (TH)

(56) Entgegenhaltungen:
- EP-A- 0 692 248
- DE-A- 2 822 125
- DE-A- 19 955 842
- DE-U- 29 817 970
- CHEMICAL ABSTRACTS, vol. 119, no. 8, 1993 Columbus, Ohio, US; abstract no. 79821a, HOYU KK: "Hair preparations for modifying curly hairs" Seite 488; Spalte 1; XP002221600 & JP 05 117134 A 14. Mai 1993 (1993-05-14)
- VALERIE PARISON: "Active delivery from Nylon particles" COSMETICS & TOILETRIES, WHEATON, IL, US, Bd. 108, Nr. 12, 1. Dezember 1993 (1993-12-01), Seiten 97-100, XP002085590 ISSN: 0361-4387

## Beschreibung

Die vorliegende Erfindung betrifft ein neues Verfahren zum dauerhaften Verformen, d.h. Dauerwellen, von menschlichen Haaren, durch das die unangenehme Geruchsentwicklung nach der Verwendung von Thiogruppen enthaltenden Reduktionsmitteln auf dauergewelltem Haar weitgehend beseitigt wird.

Es ist ein seit langem bekanntes, jedoch nach wie vor ungelöstes Problem, daß dauergewelltes Haar auch nach erfolgter Fixierung bzw. Neutralisation noch einen als unangenehm empfundenen Geruch nach dem als Verformungsmittel verwendeten Reduktionsmittel aufweist, obwohl das Haar nach der Fixierung üblicherweise mit Parfum enthaltenden Nachbehandlungsmitteln wie Shampoos, Spülungen, Kuren, etc. behandelt wird.

Es wurde auch schon vorgeschlagen, die Parfumierung von Dauerwellpräparaten und Fixiermitteln hierfür dadurch zu verbessern, daß das Parfum der entsprechenden Zusammensetzung erst unmittelbar vor der Anwendung zugesetzt wird (DE 36 40 748 C1).
Doch auch dadurch läßt sich das Problem nicht lösen.

JP5117134 beschreibt ein Verfahren zum dauerhaften Verformen von menschlichen Haaren, wobei auf das Haar in an sich bekannter Weise eine wässrige Reduktionsmittelzusammensetzung aufgebracht, anschliessend mit einer mindestens ein Oxidationsmittel enthaltenden Zusammensetzung fixiert wird (Siehe Zusammenfassung), dadurch gekennzeichnet, dass diese Zusammensetzung mit Parfum beladene Polyamidteilchen, von denen mindestens 90 Gew.-% eine Teilchengrösse im Bereich von 0,5 bis 50 Mikron aufweisen, enthält (Siehe Absätze [0011,0012,0015]).

Es wurde nun gefunden, und das ist Gegenstand der vorliegenden Erfindung, daß sich eine weitgehende und auch dauerhafte Entfernung des Reduktionsmittelgeruchs aus dem dauergewellten Haar dadurch erreichen läßt, wenn das verformte Haar nach dem Ausspülen des Reduktionsmittels mit einer mindestens ein Oxidationsmittel, insbesondere Wasserstoffperoxid, vorzugsweise in einer Konzentration von etwa 0,05 bis 6 Gew.-% enthaltenden Zusammensetzung fixiert bzw. neutralisiert wird, die 1 bis 35 Gew.-% Parfum beladene Polyamidteilchen, von denen mindestens 90 Gew.-% eine Teilchengröße im Bereich von 0,5 bis 50 Mikron, vorzugsweise etwa 1 bis etwa 25 Mikron, vor allem etwa 2 bis etwa 20 Mikron, aufweisen, enthält.
Die Bestimmung der Teilchengröße erfolgt auf dem Fachmann geläufige Weise, beispielsweise durch Sieb- oder Sedimentationsanlayse.
Die Art des verwendeten Parfumöls ist an sich zweitrangig; im Prinzip eignet sich jedes bekannte und in kosmetischen Mitteln eingesetzte Parfum für diesen Zweck.

Die Herstellung der parfumbeladenen Polyamidteilchen erfolgt durch Lösen des Parfumöls in einem geeigneten Lösungsmittel wie Wasser, Ethanol, n-Hexan etc. Diese Lösung wird unter Rühren mit dem Polyamidpulver vermischt.
Anschließend wird das Lösungsmittel, je nach Flüchtigkeit der Parfumbestandteile, unter geringer Erwärmung (etwa 25 bis maximal etwa 50°C), und/oder Vakuum entfernt.
Das Beladen von Polyamidpulver mit aktiven Materialien ist grundsätzlich bekannt und beispielsweise bei V. Parison, Cosmetics & Toiletries, Vol. 108 (Dezember 1993), S. 97-100, beschrieben.
Als Grundlage geeignete Polyamidpulver sind beispielsweise die unter den Handelsnamen "Orgasol^{R}" und "Nylon^{R}" bekannten Produkte, deren generische Bezeichnungen "Polyamide 6" oder "Polyamide 12" lauten.

Der bevorzugte Anteil derselben in der Fixier- bzw. Neutralisationszusammensetzung liegt zwischen etwa 0,1 bis etwa 10, vorzugsweise etwa 0,25 bis etwa 7,5, insbesondere etwa 0,5 bis etwa 5 Gew.-% der Zusammensetzung.

Die Menge an Parfum, mit denen die Polyamidpulverteilchen beladen sind, ist ebenfalls variabel und ist insbesondere von der Art des Parfums, der Teilchengröße des Polyamidpulvers sowie den Absorptionsbedingungen abhängig.

Sie kann zwischen etwa 1 bis etwa 35, vorzugsweise etwa 5 bis etwa 25, insbesondere etwa 10 bis etwa 20 Gew.-%, Parfum an beladenen Teilchen liegen.

Die im ersten Schritt des erfindungsgemäßen Verfahrens verwendeten Verformungsmittel enthalten in der Regel eine reduzierende Thioverbindung. Bevorzugt sind Thioglykolsäure und Thiomilchsäure sowie deren Salze, insbesondere die Ammonium- und Ethanolaminsalze.

Weitere einsetzbare Thioverbindungen sind insbesondere Cystein bzw. dessen Hydrochlorid, Homocystein, Cysteamin, N-Acetylcystein, Thioglycerin, Ethandiolmonothioglykolat, 1,2-Propylenglykolmonoglykolat (vgl. auch WO-A 93/1791), 1,3-Propandiolmonothioglykolat bzw. das daraus resultierende Isomerengemisch, 1,3-Butandiol- und 1,4-Butandiolmonothioglykolat bzw. deren Isomerengemische, Polyethylenglykol- wie Di-, Tri- und Tetraethlenglykolmonothioglykolate, Glycerinmonothiolactate und weitere Thiosäuren sowie deren Ester und Gemische derselben.

Auch die Verwendung anorganischer reduzierender Schwefelverbindungen wie Natriumhydrogensulfit ist prinzipiell möglich.

Der Gesamtgehalt an Reduktionsmitteln in der Verformungs-Zusammensetzungen beträgt üblicherweise 2,5 bis etwa 15 Gew.-%, berechnet auf freie Thioglykolsäure als Bezugssubstanz.

Die Reduktionsmittel enthaltenden Dauerwellpräparate können, falls erforderlich, einen Gehalt an Alkalisierungsmitteln aufweisen. Die Menge ist abhängig vom reduzierenden Wirkstoff und dem angestrebten pH-Wert der Zusammensetzung. Vorzugsweise enthält die Reduktionsmittel-Zusammensetzung etwa 0,1 bis etwa 5, insbesondere etwa 0,5 bis etwa 2,5 Gew.-% desselben.

Bevorzugte Alkalisierungsmittel sind Ammoniumcarbamat, Ammoniak und/oder Ammonium(bi)carbonat. Es wird die Einstellung eines pH-Wertes im Bereich zwischen etwa 6,5 und etwa 9,5, vorzugsweise etwa 7 bis 8,5, angestrebt.

Die im erfindungsgemäßen Verfahren zum Einsatz kommenden Dauerwellmittel sowie auch die Fixier- bzw. Nachbehandlungsmittel enthalten vorzugsweise auch Tenside. Deren Anteil liegt bei etwa 0,1 bis etwa 10, insbesondere etwa 1 bis etwa 5 Gew.-% der Gesamtzusammensetzung.

Sowohl bei den in den Reduktionsmittel-Zusammensetzungen als auch bei den in den Fixiermitteln eingesetzten Tensiden handelt es sich vorzugsweise um die bekannten anionaktiven Produkte, die gegebenenfalls auch in Kombination mit nichtion ischen Tensiden zum Einsatz gelangen.

Geeignete anionische Tenside sind insbesondere die bekannten Alkylethersulfate und -carbonsäuren, insbesondere in Form ihrer Alkalisalze, sowie Eiweiß-Fettsäure-Kondensate.

Geeignete nichtionische Tenside sind vorzugsweise C₈-C₁₈-Fettalkoholpolyglykolether, Fettsäurepolyglykolester, Fettsäurealkanolamide, Aminoxide und vor allem C₈-C₁₈-Alkylpolyglucoside.

Es können auch amphotere Tenside wie die bekannten Betaine und Amidobetaine sowie, insbesondere in kationischen Fixierungen, kationaktive Tenside wie quaternäre Ammoniumverbindungen eingesetzt werden.

Ein weiterer wünschenswerter Bestandteil der im erfindungsgemäßen Verfahren verwendeten Reduktionsmittel-Zusammensetzungen ist ein C₃-C₈-Alkandiol bzw. dessen Ether, insbesondere Mono-C₁-C₃-alkylether.

Bevorzugte Substanzen sind in diesem Zusammenhang 1,2- und 1,3-Propandiol, 1-Methoxypropanol(-2), 1-Ethoxypropanol(-2), 1,3- und 1,4-Butandiol, Diethylenglykol und dessen Monomethyl- und Monoethylether sowie Dipropylenglykol und dessen Monomethyl- und Monoethylether.

Der Anteil dieser Diole liegt vorzugsweise zwischen 0,5 und 30, vorzugsweise etwa 1 bis etwa 15, insbesondere etwa 1 bis etwa 10 Gew.-% der Reduktionmittel-Zusammensetzung.

Neben den C₃-C₆-Alkandiolen bzw. deren Ethern können zusätzlich auch Monoalkohole wie Ehtanol, Propanol-1, Propanol-2 sowie Polyalkohole wie Glycerin und Hexantriol, Ethylcarbitol, Benzylalkohol, Benzyloxyethanol sowie Propylencarbonat (4-Methyl-1,3-dioxolan-2-on), N-Alkylpyrrolidone und Harnstoff Verwendung finden.

Weitere mögliche Bestandteile sind kationische, anionische, nichtionische und amphothere Polymere, vorzugsweise in einer Menge von etwa 0,1 bis etwa 5, insbesondere etwa 0,25 bis 2,5 Gew.-% der Gesamtzusammensetzung des Wellmittels.

Die erfindungsgemäß eingesetzten Mittel können selbstverständlich alle in Dauerwellmitteln üblichen Stoffe enthalten, auf deren detaillierte Aufzählung hier verzichtet wird, und als wäßrige Lösungen, Emulsionen, Cremes, Schäume etc. vorliegen.

Es kann sich dabei um einphasige Produkte oder um in getrennten Verpackungen untergebrachte Zusammensetzungen handeln, die bei der Anwendung vereinigt werden, wie sie z.B. in der DE-C 43 04 828 beschrieben sind.

Zur Vermeidung von Wiederholungen wir hierzu auf den Stand der Technik verwiesen, wie er beispielsweise in "Ullmanns's Encyclopedia of Industrial Chemistry", Vol A12 (1986), S. 599 bis 591, sowie insbesondere in der Monographie von K. Schrader, "Grundlagen und Rezepturen der Kosmetika", 2. Aufl. (1989, Hüthig-Verlag), S. 823 bis 840, sowie in dem Übersichtsartikel von D. Hollenberg et al. in "Seifen-Öle-Fette-Wachse!, 117 (1991), S. 81-87, beschrieben ist.

Die dort geoffenbarten Zusammensetzungen und Einzelbestandteile, auf die ausdrücklich Bezug genommen wird, können auch im Rahmen der vorliegenden Erfindung verwendet werden.

Falls erwünscht, kann vor dem Auftrag des Reduktionsmittels noch ein Vorbehandlungsmittel appliziert werden, wie es beispielsweise in der DE-A 37 40 926 beschrieben ist. Nach dem Aufbringen dieses Vorbehandlungsmittels wird das Haar aufgewickelt und die Reduktionsmittel-Zusammensetzung aufgetragen. Nach etwa 15- bis 30-minütiger Einwirkung und Spülung erfolgt die Fixierung mit den bekannten Peroxid- oder Bromat-Zusammensetzungen, denen das mit Parfum beladene Polyamidpulver zugesetzt ist.

Bevorzugt sind Wasserstoffperoxid-Zusammensetzungen, wobei der Gehalt an H₂O₂ etwa 0,05 bis 6 Gew.-%, vorzugsweise etwa 0,1 bis 5, insbesondere etwa 0,5 bis 4 Gew.-% beträgt.

Ebenso kann selbstverständlich auch eine an sich bekannte Zwischenbehandlung zwischen Reduktions- und Neutralisierungsphase erfolgen.

Die folgenden Beispiele illustrieren die Erfindung.

### Beispiel 1

### Dauerwelle für normales Haar

| | |
|---|---|
| Ammoniumthioglykolat (50%-ig) | 21,60 (Gew.-%) |
| Ammoniumhydrogencarbonat | 5,00 |
| Kationisches Polymer (Polyquaternium-11) | 1,00 |
| Nichtionischer Emulgator (Hydrierter Ricinusölpolyolester) | 0,80 |
| Ethoxydiglykol | 1,50 |
| Chlorophyllin | 0,05 |
| Dimethicone Copolyol Meadowfoamate | 0,40 |
| Entschäumer, Trübungsmittel, Parfum | q.s. |
| Ammoniak pH | 8,5 |
| Wasser ad | 100,0 |

Die Dauerwellung erfolgte durch 20- bis 30-minütige Einwirkung auf das auf Lockenwickler gewickelte Haar, Ausspülen und anschließende fünfminütige Fixierung mit der folgenden Zusammensetzung:

| | |
|---|---|
| Wasserstoffperoxid | 2,5 (Gew.-%) |
| Cetylstearylalkohol | 2,0 |
| Natriumlauryethersulfat | 1,2 |
| C₁₂-C₁₄-Alkylpolyglykolether | 1,0 |
| Stabilisator | q.s. |
| Mit 20 Gew.-% Parfumöl beladenes Polyamidpulver | 3,0 |
| (ORGASOL^{R}, mittl. Teilchengröße 5 µm) | |
| Wasser ad | 100,0 |

Nach erneutem Ausspülen, Entfernen der Wickler und Trocknen wurde eine ausdrucksvolle Dauerwelle erzielt, die eine gepflegte Struktur und eine ausgezeichnete Naß- und Trockenkämmbarkeit zeigte.

Das Haar wies keinen unangenehmen Geruch nach Thioverbindungen auf.
Eine mit einer identischen Zusammensetzung, die jedoch anstelle des mit Parfum beladenen Polyamidpulvers 0,6 Gew.-% des Parfums alleine enthielt, ergab nach der Fixierung einen unangenehmen Geruch.

### Beispiel 2

### Zweiphasen-Produkt (Neutral-Dauerwelle)

| **Teil A:** | |
|---|---|
| Ammoniumhydrogencarbonat | 4,5 (g) |
| 1,2-Propandiol | 1,0 |
| Dimethicone Copolyol Meadowfoamate | 0,4 |
| Kationisches Polymer (Polyquaternium-22) | 1,0 |
| Cocoamidopropylbetain | 1,0 |
| Nichtionischer Lösungsvermittler | 0,8 |
| Parfum, Trübungsmittel | q.s. |
| Wasser ad | 72,0 |
| eingestellt mit NH₃ auf pH 8,6 | |

| **Teil B:** | |
|---|---|
| Ammoniumthioglykolat, 70%-ig | 18,0 (g) |
| Thiomilchsäure | 2,0 |
| 1,2-Propandiol | 0,5 |
| Wasser ad | 28,0 |
| eingestellt mit NH₃ auf pH 5,5 | |

Die Teilzusammensetzungen A und B wurden getrennt in ein bekanntes Zweikammerbehältnis eingebracht und unmittelbar vor der Anwendung auf das Haar durch Zerstören der Trennwand vereinigt, wobei ein Produkt mit einem pH-Wert von 7,4 erhalten wurde.
Die Dauerwellung erfolgte entsprechend dem in Beispiel 1 beschriebenen Verfahren.

Das Fixiermittel besaß folgende Zusammensetzung:

| | |
|---|---|
| Wasserstoffperoxid | 4,0 (Gew.-%) |
| Quaternium-36 | 2,0 |
| Citronensäure | 0,2 |
| Acetanilid | 0,2 |
| Mit 15 Gew.-% Parfumöl beladenes Polyamidpulver | 3,0 |
| (ORGASOL^{R}, mittl. Teilchengröße 10 µm) | |
| Wasser ad | 100,0 |

Es wurde ein nach dem Trocknen weitgehend und dauerhaft von unangenehmem Geruch freies, dauergewelltes Haar erhalten.

Ersatz des parfumbeladenen Polyamidpulvers durch 0,45% des gleichen freien Parfums ergab einen unangenehmen Geruch nach Schwefelverbindungen.

Im folgenden werden noch zwei Beispiele für im Rahmen der Erfindung zu verwendende Fixiermittel für Dauerwellen gegeben:

### Beispiel 3

### Fixiermittel

| | |
|---|---|
| Wasserstoffperoxid (50%-ig) | 4,8 (Gew.-%) |
| Dimethicone Copolyol | 0,1 |
| Natriumlaurylethersulfat (25%-ig) | 2,5 |
| NaH₂PO₄ | 0,2 |
| Mit 10 Gew.-% Parfumöl beladenes Polyamidpulver (Nylon^{R} 12; mittl. Teilchengröße 25 µm) | 2,5 |
| Lösungsvermittler, Stabilisator | q.s. |
| Phosphorsäure pH | 3,0 |
| Wasser ad | 100,0 |

### Beispiel 4

| | |
|---|---|
| Wasserstoffperoxid | 2,5 (Gew.-%) |
| Lauryldimethylaminoxid | 1,1 |
| Mit Parfumöl beladene Polyamidteilchen | 5,0 |
| (mittl. Teilchengröße 20 µm, Nylon^{R} 12;) | |
| Citronensäure ad pH | 3,0 |
| Wasser ad | 100,0 |

Auch bei Anwendung dieser Fixierungen wurde ein dauerhaft angenehm riechendes, dauergewelltes Haar erhalten.

## Patentansprüche

1. Verfahren zum dauerhaften Verformen von menschlichen Haaren, wobei auf das Haar in an sich bekannter Weise eine wäßrige Reduktionsmittelzusammensetzung aufgebracht und nach erfolgter Einwirkung und gegebenenfalls Zwischenbehandlung ausgespült und anschließend mit einer mindestens ein Oxidationsmittel enthaltenden Zusammensetzung fixiert wird, **dadurch gekennzeichnet, daß** diese letzte Zusammensetzung Parfum beladene Polyamidteilchen, von denen mindestens 90 Gew.-% eine Teilchengröße im Bereich von 0,5 bis 50 Mikron aufweisen, enthält wobei die Menge an Parfum, mit denen die Teilchen beladen sind, 1 bis 35 Gew.-% Parfum an beladenen Teilchen beträgt.

2. Verfahren nach Anspruch 1, **dadurch gekennzeichnet, daß** die Teilchengröße von mindestens 90 Gew.-% der Polyamidteilchen im Bereich von 1 bis 25 Mikron liegt.

3. Verfahren nach Anspruch 1 und/oder 2, **dadurch gekennzeichnet, daß** die Fixiermittelzusammensetzung 0,05 bis 6 Gew.-% Wasserstoffperoxid enthält.

## Claims

1. Process for the permanent waving of human hair, whereby an aqueous reducing agent is applied onto the hair in a manner known per se, left to process, then optionally treated with an intermediate treatment, rinsed and subsequently neutralized with a composition comprising at least one oxidizing agent, **characterized in that** this neutralizing composition comprises polyamide particles charged with perfume, of which at least 90 % have a particle size from 0.5 to 50 microns, whereby the amount of perfume with which the particles are charged is 1 to 35% by wt. of perfume compared with the charged particle.

2. Process according to claim 1, wherein at least 90 % by weight of the polyamide particles have a particle size from 1 to 25 microns.

3. Process according to claim 1 and/or 2, wherein the neutralizing composition comprises 0.05 % to 6 % by weight, calculated to the total composition, of hydrogen peroxide.

## Revendications

1. Procédé de mise en forme durable des cheveux humains, dans lequel une composition aqueuse d'agent réducteur est appliquée, de manière connue en soi, sur les cheveux, et est rincée après son action et un éventuel traitement intermédiaire et finalement fixée avec une composition qui comporte au moins un agent oxydant, **caractérisé en ce que** cette dernière composition contient des particules de polyamide chargées en parfum, dont au moins 90 % en poids présentent une taille allant de 0,5 à 50 micromètres, la quantité de parfum dont les particules sont chargées représentant de 1 à 35 % en poids de parfum par rapport aux particules chargées.

2. Procédé selon la revendication 1, **caractérisé en ce que** la taille d'au moins 90 % des particules de polyamide est comprise entre 1 et 25 micromètres.

3. Procédé selon les revendications 1 et/ou 2, **caractérisé en ce que** la composition d'agent de fixation contient de 0,05 à 6 % en poids de peroxyde d'hydrogène.
